# EUROPÄISCHE PATENTSCHRIFT

(11) **EP 0 731 798 B1**
(45) Veröffentlichungstag und Bekanntmachung des Hinweises auf die Patenterteilung: **13.01.1999**
(21) Anmeldenummer: 95901428.3
(22) Anmeldetag: 24.11.1994
(51) Int. Cl.: C07D 307/92

(54) **VERFAHREN ZUR HERSTELLUNG VON 8$g(a),12-OXIDO-13,14,15,16-TETRANORLABDAN**
PROCESS FOR PRODUCING 8ALPHA,12-OXIDO-13,14,15,16-TETRANORLABDANE
PROCEDE DE PRODUCTION DE 8ALPHA,12-OXYDO-13,14,15,16-TETRANORLABDANE

(30) Priorität: 03.12.1993 DE 4341272
(43) Veröffentlichungstag der Anmeldung: 18.09.1996
(73) Patentinhaber: Henkel Kommanditgesellschaft auf Aktien, 40191 Düsseldorf (DE)
(72) Erfinder: KNÜBEL, Georg, D-40229 Düsseldorf (DE); BOMHARD, Andreas, D-40591 Düsseldorf (DE); SCHAPER, Ulf-Armin, D-47804 Krefeld (DE); STALBERG, Theo, D-40789 Monheim (DE); MARKERT, Thomas, D-40591 Düsseldorf (DE)
(86) Internationale Anmeldenummer: EP9403891
(87) Internationale Veröffentlichungsnummer: WO9515321

(56) Entgegenhaltungen:
- WO-A-90/12793
- WO-A-93/02073
- CHEMICAL ABSTRACTS, vol. 105, no. 15, 13. Oktober 1986, Columbus, Ohio, US; abstract no. 134193k, Seite 703 ;

## Beschreibung

### Gebiet der Erfindung

Die Erfindung betrifft ein Verfahren zur Herstellung von 8α,12-Oxido-13,14,15,16-tetranorlabdan sowie die Verwendung spezieller Aluminoschichtsilikate zur Herstellung von 8α,12-Oxido-13,14,15,16-tetranorlabdan.

### Stand der Technik

8α,12-Oxido-13,14,15,16-tetranorlabdan, fortan als **Ambroxan** bezeichnet, ist ein wertvoller Ambrariechstoff, der in der Ambra, einer Stoffwechselausscheidung des Pottwals, enthalten ist (Ullmanns Encyklopädie der technischen Chemie, Band 20, Seite 283, Verlag Chemie Weinheim 1981). Synthetisch kann Ambroxan aus Sclareol durch oxidativen Seitenkettenabbau und nachfolgende Reduktion des gebildeten Lactons **(Sclareolid)** gemäß US 30 50 532 hergestellt werden. Die Überführung von Sclareolid in das geruchlose 8α,12-Dihydroxy-13,14,15,16-tetranorlabdan, fortan kurz als **Diol** bezeichnet, erfolgt in an sich bekannter Weise, beispielsweise durch Reduktion mit Lithiumaluminiumhydrid (Helv. Chim. Acta 1950, 33, 1310), mit Natriumborhydrid (Chem. Abstr. 57, 7316a) oder mit Kaliumborhydrid/Lithiumchlorid-Mischungen (Chem. Abstr. 94, 15913q).

Die cyclisierende Dehydratisierung des Diols zu Ambroxan kann mit sauren Katalysatoren, beispielsweise p-Toluolsulfonsäure, p-Toluolsulfonsäurechlorid, katalytischen Mengen an Schwefelsäure sowie sauren Ionenaustauschern in verschiedenen Lösungsmitteln, beispielsweise Toluol, Hexan, Pyridin, Tetrahydrofuran oder Methanol, vorzugsweise bei Siedetemperatur, durchgeführt werden.

In US 3 029 255 wird die Verwendung von β-Naphthalinsulfonsäure oder Tonerde als Dehydratisierungskatalysatoren bei der Herstellung von Ambroxan beschrieben. Bei diesem Verfahren werden neben Verharzungsprodukten und Olefinen weitere Nebenprodukte erhalten, so daß die Ausbeute an Ambroxan weniger als 77 % beträgt.

JP-A-86/33184 (Takasago) beschreibt ein Verfahren zur Herstellung von Ambroxan, bei der die Cyclisierung der Diolvorstufe durch spezielle Katalysatoren induziert wird. Bei diesen Katalysatoren handelt es sich um säure-beladene aktive Weißerde, Tonerde oder Kieselerde. Als Säuren werden dabei insbesondere Schwefelsäure, Phosphorsäure und Polyphosphorsäure offenbart. Das Takasago-Verfahren hat jedoch Nachteile bezüglich
a) des Umsatzes an Edukt, d. h. an eingesetztem Diol und/oder
b) der Bildung an Dehydratisierungsprodukten (Nebenprodukten) und/oder
c) der Stereoselektivität der Ringschlußreaktion (Ausmaß der iso-Ambrox-Bildung).

Die genannten Nachteile des Takasago-Verfahrens werden durch das in der jüngeren Anmeldung WO 90/12793 beschriebene Verfahren der Anmelderin vermieden. Das Verfahren gemäß WO 90/12793 erfordert jedoch relativ hohe Reaktionstemperaturen und relativ große Mengen an Katalysator. Darüber hinaus läßt sich der spezielle HCl-beladene Katalysator nicht ohne weiteres wiederverwenden.

### Beschreibung der Erfindung

Die Aufgabe der vorliegenden Erfindung bestand in der Entwicklung eines Verfahrens zur Herstellung von Ambroxan durch cyclisierende Dehydratisierung der Diol-Vorstufe, das die Nachteile der aus dem Stand der Technik bekannten Verfahren vermeidet.

Überraschenderweise wurde nun gefunden, daß Ambroxan mit hoher Reinheit und Ausbeute hergestellt werden kann, wenn man die Diol-Vorstufe in einem Lösungsmittel und in Gegenwart von Aluminoschichtsilikaten mit einer Säurebeladung von 100 bis 300 mval/100g cyclisierend dehydratisiert.

Unter dem Begriff "Säurebeladung" ist im Rahmen der vorliegenden Erfindung derjenige Anteil des Gesamtsäuregehalts der Aluminoschichtsilikate zu verstehen, der nur locker an den Feststoff gebunden ist und sich nach Elution mit Wasser analytisch durch Titration erfassen läßt. In diesem Zusammenhang sei angemerkt, daß die K-Katalysatoren darüber hinaus einen Anteil an Säure enthalten, welcher ionisch an das Gerüst des Katalysators gebunden ist und in wäßriger Dispersion nicht abdissoziiert.

Gegenstand der Erfindung ist ein Verfahren zur Herstellung von 8α,12-Oxido-13,14,15,16-tetranorlabdan durch Dehydratisierung von 8α,12-Dihydroxy-13,14,15,16-tetranorlabdan (Diol), wobei man 8α,12-Dihydroxy-13,14,15,16-tetranorlabdan in Gegenwart von Aluminoschichtsilikaten mit einer Säurebeladung von 100 bis 300 mval/100g in einem Lösungsmittel cyclisierend dehydratisiert.

Das erfindungsgemäße Verfahren hat den Vorteil, daß der Katalysator nur in relativ geringen Mengen eingesetzt wird und daß er weitgehend wiederverwendet werden kann. Darüber hinaus erfordert die Durchführung des erfindungsgemäßen Verfahrens nur sehr moderate Temperaturen, so daß durch diese besonders schonende Art der Herstellung die Gefahr der Bildung olfaktorisch unerwünschter Nebenprodukte vermindert ist. Die genannten Vorteile des erfindungsgemäßen Verfahrens gehen nicht zu Lasten der aus der WO 90/12793 bekannten guten Stereoselektivität der Ringschlußreaktion. Dies bedeutet, daß der Anteil des Produkts an den Ambroxan-Isomeren 8-epi- bzw. 9-epi-Ambroxan in derselben Größenordnung liegt, wie bei dem Verfahren gemäß WO 90/12793.

Aluminoschichtsilikate sind Mineralien mit silikatischer Grundstruktur, in denen miteinander über Dipol-Dipol-Wechselwirkungen und Wasserstoffbrückenbindungen verknüpfte Silikatschichten mit teilweise eingelagerten Aluminium³⁺-Ionen vorliegen, und wobei diese zweidimensional unendlichen anionischen Silikatschichten über Kationen einer Zwischenschicht elektrostatisch vernetzt sind. Struktur und Zusammensetzung derartiger Schichtsilikate sind aus dem Stand der Technik bekannt und können der einschlägigen Literatur entnommen werden. Beispiele für Aluminoschichtsilikate sind Talk sowie Tone mit Blattstruktur wie Kaolinit, Montmorillonit, Bentonite und Hectorite.

Die Menge des Dehydratisierungskatalysators, d. h. des säurebeladenen Aluminoschichtsilikates, ist an sich nicht kritisch. Üblicherweise wird der Katalysator in dem erfindungsgemäßen Verfahren jedoch in einer Menge von - bezogen auf das Diol - 5 bis 80 Gew.-% eingesetzt. Die bevorzugte Menge liegt im Bereich von 15 bis 35 Gew. -%.

Die Ringschlußreaktion wird bei Temperaturen im Bereich von 20 bis 110 °C durchgeführt. Dabei ist der Bereich von 40 bis 70 °C, insbesondere von 40 bis 50 °C, bevorzugt.

In dem erfindungsgemäßen Verfahren setzt man als Dehydratisierungskatalysator säurebeladenes Aluminoschichtsilikat ein. Die Säurebeladung liegt erfindungsgemäß im Bereich von 100 bis 300 mval/100 g. Besonders bevorzugt ist jedoch eine Säurebeladung im Bereich von 130 bis 180 mval/100 g.

Die Art der Säure ist an sich keinen speziellen Einschränkungen unterworfen. Besonders bevorzugt sind jedoch Halogenwasserstoffsäuren, insbesondere HCl, sowie Schwefelsäure und Phosphorsäure.

Besonders bevorzugt als Dehydratisierungskatalysatoren sind im Sinne der vorliegenden Erfindung säurebeladene Montmorillonite. In einer bevorzugten Ausführungsform der vorliegenden Erfindung werden dabei sogenannte K-Katalysatoren, die dem Fachmann z. b. aus Nachr. Chem. Tech. Lab. 1985 (33) Nr. 3, S. 202, bekannt sind, mit der oben beschriebenen Säurebeladung eingesetzt. Erfindungsgemäß geeignet sind dabei insbesondere die kommerziell erhältlichen Typen KSF und KSF/O. Die K-Katalysatoren können dabei allein oder in Kombination miteinander eingesetzt werden.

Als Dehydratisierungskatalysatoren können im Rahmen der vorliegenden Erfindung einerseits solche Aluminioschichtsilikate eingesetzt werden, die herstellungsbedingt bereits die erforderliche kritische Säurebeladung im Bereich von 100 bis 300 mval/100 g aufweisen, wie es z. B. für die K-Katalysatoren vom Typ KSF und KSF/O der Fall ist. Es ist jedoch auch möglich, solche Aluminoschichtsilikate als Dehydratisierungskatalysatoren einzusetzen, die herstellungsbedingt zunächst eine geringere Säurebeladung aufweisen, die jedoch nachträglich mit soviel Säure beladen wurden, daß ihre Säurebeladung im obengenannten kritischen Bereich liegt.

Das Diol wird üblicherweise in wasserfreier Form eingesetzt. Es ist jedoch auch möglich, ein Diol technischer Qualität mit einem Wassergehalt bis zu ca. 2 Gew. -% einzusetzen.

Ein weiterer Gegenstand der vorliegenden Erfindung ist die Verwendung von Aluminoschichtsilikaten mit einer Säurebeladung von 100 bis 300 mval/100 g, vorzugsweise 130 bis 180 mval/100 g, zur Herstellung von 8α,12-Oxido-13,14,15,16-tetranorlabdan durch cyclisierende Dehydratisierung von 8α,12-Dihydroxy-13,14,15,16-tetranorlabdan.

Geeignete Lösungsmittel für die cyclisierende Dehydratisierung des Diols sind beispielsweise Toluol und/oder Xylol.

Das während der Dehydratisierung gebildete Wasser kann beispielsweise durch azeotrope Destillation aus dem Reaktionsgemisch entfernt werden. Nach beendeter Dehydratisierung wird das Reaktionsgemisch in an sich bekannter Weise aufgearbeitet.

Das erfindungsgemäße Verfahren kann batchweise oder kontinuierlich durchgeführt werden. Die kontinuierliche Arbeitsweise, z. B. in einem Festbettreaktor, bietet dabei den zusätzlichen Vorteil, daß es keiner speziellen Abtrennung des stückigen Katalysators bedarf.

Die folgenden Beispiele dienen der Erläuterung der Erfindung und sind nicht einschränkend zu verstehen.

### Beispiele

### 1. Allgemeines

### 1.1. Verwendete Substanzen

- **Diol:**: 8α,12-Dihydroxy-13,14,15,16-tetranorlabdan
- **Kat-1:**: Bentonit aktiv, Typ B (Fa. Erbsloh) Säuregehalt: 80 mval H₂SO₄/100 g
- **Kat-2:**: Siliciumdioxid ("Kieselgel 60", Korngröße < 0,063 mm; Fa. Merck), beladen mit Schwefelsäure; Säuregehalt: 200 mval H₂SO₄/100 g
- **Kat-3:**: Aluminiumoxid neutral (Fa. Riedel de Haen), beladen mit Schwefelsäure; Säuregehalt: 200 mval H₂SO₄/100 g
- **Kat-4:**: Aluminiumoxid neutral (Fa. ICN), beladen mit Salzsäure; Säuregehalt: 14 mval HCl/100 g
- **Kat-5:**: Katalysator KSF/O (Fa. Süd-Chemie AG); Säuregehalt: ca. 155 mval H₂SO₄/100 g

### 1.2. Analytik

Zur Quantifizierung der Produkte wurde die gaschramotographische Analyse herangezogen (50 m WG11- Quartz-Kapillare; Injektortemperatur: 220 °C; Detektortemperatur: 250 °C; Ofentemperatur: 80 -> 220 °C bei einer Aufheizgeschwindigkeit von 8 °C/min; Trägergas: Stickstoff; Druck: 20 psi).

### 2. Versuchsbeschreibungen

### 2.1. Herstellung von Ambroxan nach da Verfahren gemäß JP-A-86/33184

### a) Vergleichsbeispiel V1

51 g n-Heptanol sowie 2,54 g Diol wurden in einem 200-ml-Kolben vorgelegt und das Diol unter Rühren gelöst. Nach dem Zufügen von 0,13 g Katalysator Kat-1 wurde auf einen Druck von 10 mmHg evakuiert und das System bei diesem Druck langsam auf 40 °C erwärmt. Anschließend wurde 3 Stunden bei dieser Temperatur gerührt. Der Katalysator wurde abfiltriert, das Filtrat mit wäßriger Natriumcarbonatlösung gewaschen, das n-Heptanol anschließend im Vakuum abdestilliert.

Die Ausbeute an Ambroxan war mit 7,7 % - bezogen auf eingesetztes Diol - sehr gering; der Anteil an Dehydrierungsprodukten wurde zu 2,5 % - bezogen auf Ambroxan - bestimmt (vergl. Tabelle 1).

### b) Vergleichsbeispiel V2

In einem 2-l-Kolben wurde eine Lösung von 25,4 g Diol in 500 g Xylol zu einer Aufschlämmung von 5 g Katalysator Kat-1 in 200 ml Xylol gegeben. Es wurde auf einen Druck von 50 mmHg evakuiert und das System bei diesem Druck langsam erhitzt. Anschließend wurde 4 Stunden unter Rückfluß gekocht. Der Katalysator wurde anschließend abfiltriert, das Filtrat mit wäßriger Natriumcarbonatlösung gewaschen und schließlich das Xylol abdestilliert.

Die Ausbeute an Ambroxan betrug 32,2 % - bezogen auf eingesetztes Diol; das Produkt enthielt 6,0 % - bezogen auf Ambroxan - an Dehydrierungsprodukten; der Anteil an iso-Ambroxan betrug 1,0 % - bezogen auf eingesetztes Diol (vergl. Tabelle 1).

### Vergleichsbeispiele V3 bis V5

Die für Vergleichsbeispiel V2 beschriebenen Operationen wurden wiederholt, wobei folgende Parameter variiert wurden: Temperatur sowie Art und Menge des Katalysators. Für Einzelheiten sei auf Tabelle 2 verwiesen.

Die bei den Versuchen V3 bis V5 ermittelten Daten können Tabelle 1 entnommen werden.

### 2.2. Herstellung von Ambroxan gemäß der Erfindung

### Beispiel B1

600 g technisches Diol (Anteil an reinem Diol : 90 %) wurden in 600 ml Toluol gelöst und mit 120 g KSF/O versetzt. Unter Rühren wurde 4 Stunden auf 50 °C erwärmt. Im Anschluß filtrierte man die Reaktionslösung ab und extrahierte unter Rückfluß den Katalysator mit 300 ml Toluol. Die vereinigten organischen Phasen wurden dann mit 1 %iger Salpetersäure, 1 %iger Natronlauge und abschließend 10 %iger Natriumsulfat-Lösung gewaschen. Im Anschluß wurde im Vakuum bis zur Trockene eingeengt.

Das Rohprodukt wurde gaschromatographisch analysiert. Die dabei ermittelten Daten können Tabelle 1 entnommen werden.

Der zurückgewonnene Katalysator wurde erneut zur Diol-Cyclisierung eingesetzt. Dabei konnten keinerlei Nachteile in Bezug auf Ausbeute bzw. Nebenproduktbildung festgestellt werden.

### 3. Diskussion

Der Vergleich des erfindungsgemäßen Beispiels B1 mit den Vergleichsbeispielen V1 bis V5 zeigt, daß in bezug auf Ausbeuten und Stereoselektivität deutliche Unterschiede bestehen. Der besseren Übersicht halber sind die o. g. Daten nochmals in Tabelle 1 zusammengestellt; wichtige Versuchsparameter der Beispiele V1 bis V5 und B1 sind in Tabelle 2 zusammengestellt.

**Tabelle 1**

| Beispiel | Ambroxan^{a)} | iso-Ambroxan^{a)} | | Dehydratisierungsprodukte^{b)} |
|---|---|---|---|---|
| | | 8-epi | 9-epi | |
| V1 | 7,7 % | n.b.^{c)} | n.b. | 2,5 % |
| V2 | 32,2 % | 0,9 % | 0,1 % | 6,0 % |
| V3 | 68,1 % | 3,0 % | 0,1 % | 17,3 % |
| V4 | 66,3 % | 0,8 % | 0,05 % | 16,1 % |
| V5 | k.U.^{d)} | n.b. | n.b. | n.b. |
| B1 | 98,7 % | < 0,05 % | < 0,05 % | 2,7 % |

| | | | | |
|---|---|---|---|---|
| ^{a)} Gew. -% bezogen auf eingesetztes Diol | | | | |
| ^{b)} Gew.-% bezogen auf Ambroxan | | | | |
| ^{c)} n. b. = nicht bestimmt | | | | |
| ^{d)} k. U. = keine Umsetzung | | | | |

**Tabelle 2**

| Beispiel | Temperatur | Katalysator | | |
|---|---|---|---|---|
| | | Art | Menge^{e)} | Säurebeladung^{f)} |
| V1 | 40 °C | Kat-1 | 5 % | 80 |
| V2 | 130 °C | Kat-1 | 20 % | 80 |
| V3 | 130 °C | Kat-2 | 5 % | 200 |
| V4 | 130 °C | Kat-3 | 5 % | 200 |
| V5 | 50 °C | Kat-4 | 60 % | 16 |
| B1 | 50 °C | Kat-5 | 20 % | 155 |

| | | | | |
|---|---|---|---|---|
| ^{e)} Gew. -% bezogen auf eingesetztes Diol | | | | |
| ^{f)} in mval/100 g Katalysator | | | | |

## Patentansprüche

1. Verfahren zur Herstellung von 8α,12-Oxido-13,14,15,16-tetranorlabdan durch Dehydratisierung von 8α,12-Dihydroxy-13,14,15,16-tetranorlabdan (Diol), **dadurch gekennzeichnet**, daß man das Diol in Gegenwart von Aluminoschichtsilikaten mit einer Säurebeladung von 100 bis 300 mval/100 g in einem Lösungsmittel cyclisierend dehydratisiert.

2. Verfahren nach Anspruch 1, wobei man den Dehydratisierungskatalysator in einer Menge von - bezogen auf das Diol - 5 bis 80 Gew. -%, vorzugsweise 15 bis 35 Gew. -%, einsetzt.

3. Verfahren nach Anspruch 1 oder 2, wobei man die Dehydratisierung des Diols bei Temperaturen im Bereich von 40 bis 70 °C durchführt.

4. Verfahren nach einem der Ansprüche 1 bis 3, wobei man ein Aluminoschichtsilikat mit einer Säurebeladung von 130 bis 180 mval/100 g einsetzt.

5. Verfahren nach einem der Ansprüche 1 bis 4, wobei man Schwefelsäure-beladene Aluminoschichtsilikate einsetzt.

6. Verfahren nach einem der Ansprüche 1 bis 5, wobei man als Dehydratisierungskatalysator säurebeladene Montmorillonite einsetzt.

7. Verfahren nach einem der Ansprüche 1 bis 6, wobei man als Dehydratisierungskatalysator einen K-Katalysator, insbesondere KSF und/oder KSF/O, einsetzt.

8. Verwendung von Aluminoschichtsilikaten mit einer Säurebeladung von 100 bis 300 mval/100 g, vorzugsweise 130 bis 180 mval/100 g, zur Herstellung von 8α,12-Oxido-13,14,15,16-tetranorlabdan durch cyclisierende Dehydratisierung von 8α,12-Dihydroxy-13,14,15,16-tetranorlabdan.

## Claims

1. A process for the production of 8α, 12-oxido-13,14,15,16-tetranorlabdane by dehydration of 8α,12-dihydroxy-13,14,15,16-tetranorlabdane (diol), characterized in that the diol is subjected to cyclizing dehydration in a solvent in the presence of alumino layer silicates with an acid charge of 100 to 300 mval/100 g.

2. A process as claimed in claim 1, characterized in that the dehydration catalyst is used in a quantity of - based on the diol - 5 to 80% by weight and preferably 15 to 35% by weight.

3. A process as claimed in claim 1 or 2, characterized in that the dehydration of the diol is carried out at temperatures of 40 to 70°C.

4. A process as claimed in any of claims 1 to 3, characterized in that an alumino layer silicate with an acid charge of 130 to 180 mval/100 g is used.

5. A process as claimed in any of claims 1 to 4, characterized in that alumino layer silicates charged with sulfuric acid are used.

6. A process as claimed in any of claims 1 to 5, characterized in that acid-charged montmorillonites are used as the dehydration catalyst.

7. A process as claimed in any of claims 1 to 6, characterized in that a K catalyst, more especially KSF and/or KSF/O, is used as the dehydration catalyst.

8. The use of alumino layer silicates with an acid charge of 100 to 300 mval/100 g and preferably 130 to 180 mval/100 g for the production of 8α, 12-oxido-13,14,15,16-tetranorlabdane by cyclizing dehydration of 8α,12-dihydroxy-13,14,15,16-tetranorlabdane.

## Revendications

1. Procédé de production de 8α,12-oxydo-13,14,15,16-tétranorlabdane par déshydratation de 8α,12-dihydroxy-13,14,15,16-tétranorlabdane (diol), **caractérisé en ce que** l'on déshydrate le diol par cyclisation en présence d'aluminosilicates lamellaires avec une charge d'acide de 100 à 300 mEq/100 g dans un solvant.

2. Procédé selon la revendication 1, dans lequel on met en oeuvre le catalyseur de déshydratation dans une proportion (par rapport au diol) de 5 à 80 % en poids, de préférence de 15 à 35 % en poids.

3. Procédé selon la revendication 1 ou 2, dans lequel on opère la déshydratation du diol à des températures comprises dans l'intervalle de 40 à 70 °C.

4. Procédé selon une des revendications 1 à 3, dans lequel on met en oeuvre un aluminosilicate lamellaire avec une charge d'acide de 130 à 180 mEq/100 g.

5. Procédé selon une des revendications 1 à 4, dans lequel on met en oeuvre des aluminosilicates lamellaires chargés d'acide sulfurique.

6. Procédé selon une des revendications 1 à 5, dans lequel on met en oeuvre comme catalyseur de déshydratation, des montmorillonites chargées d'acide.

7. Procédé selon une des revendications 1 à 6, dans lequel on met en oeuvre comme catalyseur de déshydratation, un catalyseur K, en particulier KSF et/ou KSF/O.

8. Utilisation d'aluminosilicates lamellaires avec une charge d'acide de 100 à 300 mEq/100 g, de préférence de 130 à 180 mEq/100 g, pour la production de 8α,12-oxydo-13,14,15,16-tétranorlabdane par déshydratation cyclisante de 8α,12-dihydroxy-13,14,15,16-tétranorlabdane.
